# EUROPEAN PATENT APPLICATION

(11) **EP 4 029 421 A1**
(43) Date of publication of application: **20.07.2022**
(21) Application number: 21151298.3
(22) Date of filing: 13.01.2021
(51) Int. Cl.: A47K 13/00, A61L 2/00, A61L 2/10, A61L 2/24, E05B 1/00, E03D 9/00

(54) **USE OF POLARIZED RADIATION FOR DETECTING TOUCH ON A RADIATION EXIT WINDOW**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); Niessen, Eduard Matheus Johannes, 5656 AE Eindhoven (NL); Martens, Peter, 5656 AE Eindhoven (NL)
(74) Representative: Philips Intellectual Property & Standards

(57) **Abstract**

In a system (1) comprising a radiation body (10), a radiation arrangement (20), a detector arrangement (30) and a controller arrangement (40), the controller arrangement (40) is configured to put a UV radiation source (21) of the radiation arrangement (20) from a default state to a state of reduced radiation intensity when detection results demonstrate occurrence of a disturbance incident that is indicative of touch on a radiation exit window (11) of the radiation body (10). In particular, at least one radiation source (21) of the radiation arrangement (20) is configured to emit polarized radiation (22), and the detector arrangement (30) is configured to detect at least one of intensity of polarized radiation of the source polarization orientation and intensity of polarized radiation of orthogonal polarization orientation, wherein a change of the at least one of those intensities is taken as an indication of occurrence of a disturbance incident.

## Description

### FIELD OF THE INVENTION

The invention relates to a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body; a radiation arrangement configured to provide the radiation, comprising at least one UV radiation source configured to emit UV radiation; a detector arrangement configured to detect intensity of radiation; and a controller arrangement, functionally coupled to the radiation arrangement and the detector arrangement, configured to set a normal state of the at least one UV radiation source in which the at least one UV radiation source provides the UV radiation with an intensity at an operational level as a default, and to put the at least one UV radiation source from the default state to an adapted state in which the at least one UV radiation source provides the UV radiation with an intensity at a level that is reduced relative to the operational level after occurrence of a disturbance incident, wherein the controller arrangement is configured to determine occurrence of a disturbance incident by processing input from the detector arrangement.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element, an optical sensor and a control system. The waveguide element comprises a radiation exit window, wherein the waveguide element is i) configured to receive radiation, wherein the radiation at least comprises UV radiation, ii) configured to radiate at least part of the radiation to the exterior of the waveguide element via the radiation exit window, and iii) configured to internally reflect part of the radiation at the radiation exit window. The optical sensor is configured to sense an internal reflection intensity of the internally reflected radiation. The control system is functionally coupled to the optical sensor and is configured to reduce the intensity of the radiation as function of reaching a predetermined first threshold of a reduction of the internal reflection intensity over time.

WO 2018/215272 A1 teaches that the UV radiation is used for killing microorganisms as may be present on the radiation exit window, or for rendering the microorganisms inactive or unable to reproduce. Practical examples of microorganisms are bacteria. The disinfecting effect of using UV radiation is mainly governed by a total dose of the UV radiation. In the context of using UV radiation, it may be necessary to take specific measures when higher organisms, including human beings, may be in a position of receiving the UV radiation, which is especially the case if it is possible for the higher organisms to physically contact radiation emitting surfaces.

During operation of the known system, radiation is coupled out of the waveguide element through the radiation exit window if some object touches the window. This outcoupling means that less radiation will stay inside the waveguide element and is also referred to as frustration of the (total) internal reflection. Therefore, by using the optical sensor to monitor an internal reflection intensity of the internally reflected radiation, it is possible to detect a situation of some object touching the window, especially some object that is considerably larger than microorganisms, such as a human hand or finger. When a considerable step is observed in the signal provided by the optical sensor, it is assumed that this means that a relatively large object contacts the window. In order to ensure safety in such a situation, it may be determined that the radiation needs to be shut off, at least temporarily.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications of the invention that is the subject of WO 2018/215272 A1, it is important that external surfaces are kept in a disinfected state while situations in which the UV radiation used in the process might cause harm to higher organisms such as human beings need to be avoided.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. However, the same principle of outcoupling the radiation at contact positions would cause potentially unsafe exposure of human beings touching the waveguide element to UV radiation if it was not for the measure of detecting the touching and shutting off the radiation. In the context of the present invention, it is acknowledged that there is a need for developing useful ways of actually putting this measure to practice.

### SUMMARY OF THE INVENTION

It is an object of the invention to design a system comprising a radiation body and a radiation arrangement in such a way that a proper balance is realized between effectively obtaining disinfection results at the position of a radiation exit window of the radiation body on the one hand and protecting human beings and other higher organisms from harmful exposure to UV radiation provided by at least one UV radiation source of the radiation arrangement on the other hand. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body; a radiation arrangement configured to provide the radiation, comprising at least one radiation source including at least one UV radiation source configured to emit UV radiation, wherein at least one radiation source of the radiation arrangement is configured to emit polarized radiation; a detector arrangement configured to detect at least one of intensity of polarized radiation of the same polarization orientation as the polarized radiation emitted by the at least one radiation source and intensity of polarized radiation of orthogonal polarization orientation; and a controller arrangement, functionally coupled to the radiation arrangement and the detector arrangement, configured to set a normal state of the at least one UV radiation source in which the at least one UV radiation source provides the UV radiation with an intensity at an operational level as a default, and to put the at least one UV radiation source from the default state to an adapted state in which the at least one UV radiation source provides the UV radiation with an intensity at a level that is reduced relative to the operational level after occurrence of a disturbance incident involving a change of the at least one of intensity of polarized radiation of the same polarization orientation as the polarized radiation emitted by the at least one radiation source and intensity of polarizated radiation of orthogonal polarization orientation detected by the detector arrangement.

It follows from the foregoing that the invention is aimed at providing a functionality of the system according to which the at least one UV radiation source of the radiation arrangement is put from the default state to the adapted state when detection results obtained by means of the detector arrangement demonstrate occurrence of a disturbance incident. In particular, at least one radiation source of the radiation arrangement is configured to emit polarized radiation, and the detector arrangement is configured to detect at least one of intensity of polarized radiation of the same polarization orientation as the polarized radiation emitted by the at least one radiation source and intensity of polarizated radiation of orthogonal polarization orientation, wherein a change of the at least one of the intensities as mentioned is taken as an indication of occurrence of a disturbance incident. In this way, assuming that occurrence of a disturbance incident is indicative of the radiation exit window being contacted by a relatively large object such as a human hand or finger, the controller arrangement is configured to enable disinfection of the radiation exit window as a default, and to implement a safety measure of reducing the intensity at which UV radiation is provided whenever appropriate. The reduced level of the intensity of the UV radiation provided by the at least one UV radiation source in the adapted state may be a zero level, although this is not necessary in the context of the invention. Further, the system may be designed in any appropriate way to realize/enable restoration of the default state of the at least one UV radiation source in any possible suitable fashion after the adapted state of the at least one UV radiation source has been set, and the controller arrangement may particularly be configured to maintain the adapted state of the at least one UV radiation source only during a limited amount of time.

An insight of the invention is that by using polarized radiation for detection purposes, high detection sensitivity can be obtained, and that this can be done without compromising the disinfecting functionality of the system. In the case that the UV radiation is the polarized radiation, there is no difference of disinfecting effectiveness of the UV radiation. Irrespective of whether the UV radiation or another type of radiation is the polarized radiation, when the radiation exit window is touched by a relatively large object such as a human hand or finger, it happens that some of the polarized radiation is scattered back into the radiation body and also that a change of polarization orientation of part of the radiation is caused. Especially when this type of change is relied on for the purpose of automatically recognizing a situation of touch, the high detection sensitivity as mentioned can be realized, wherein it is to be noted that the detection sensitivity can be higher than in cases in which a change of the constitution of the radiation under the influence of touch on the radiation exit window is not envisaged.

According to a practical option, the controller arrangement is configured to determine occurrence of a disturbance incident when a value of the at least one of intensity of polarized radiation of the same polarization orientation as the polarized radiation emitted by the at least one radiation source and intensity of polarizated radiation of orthogonal polarization orientation shifts from inside a safety reference range of values to outside of the safety range of values. For example, an intensity threshold may be defined, wherein the safety reference range of values comprises values higher than the intensity threshold, or lower than the intensity threshold, as appropriate in an actual embodiment. As suggested earlier, a change of polarization orientation may be expected in respect of part of the polarized radiation under the influence of touch on the radiation exit window. Thus, it happens that the value of the intensity of polarized radiation of the same polarization orientation as the polarized radiation emitted by the at least one radiation source decreases and the value of the intensity of polarized radiation of the orthogonal polarization orientation increases. At least one of both of these effects can be measured. In respect of the option of measuring one of these effects, it is to be noted that measuring the latter effect is expected to yield the most accurate results, as the value of the intensity of polarized radiation of the orthogonal polarization orientation increases from practically zero to a certain value, i.e. increases by a very large factor.

According to another or additional option, the detector arrangement is configured to detect both intensity of polarized radiation of the same polarization orientation as the polarized radiation emitted by the at least one radiation source and intensity of polarized radiation of orthogonal polarization orientation, and the controller arrangement is configured to determine occurrence of a disturbance incident when a ratio of the respective intensities shifts from inside a safety reference range of ratios to outside of the safety range of ratios. This option may involve even higher detection sensitivity.

In practical applications of the invention, the polarized radiation emitted by the at least one radiation source of the radiation arrangement is of horizontal polarization orientation or vertical polarization orientation, and the detector arrangement is configured to detect at least one of intensity of polarized radiation of horizontal polarization orientation and intensity of polarized radiation of vertical polarization orientation. In principle, any combination of polarization orientations is possible, meaning that the invention covers all of the various options: i) the polarized radiation emitted by the at least one radiation source of the radiation arrangement is of horizontal polarization orientation and the detector arrangement is configured to detect intensity of polarized radiation of horizontal polarization orientation, ii) the polarized radiation emitted by the at least one radiation source of the radiation arrangement is of horizontal polarization orientation and the detector arrangement is configured to detect intensity of polarized radiation of vertical polarization orientation, iii) the polarized radiation emitted by the at least one radiation source of the radiation arrangement is of horizontal polarization orientation and the detector arrangement is configured to detect both intensity of polarized radiation of horizontal polarization orientation and intensity of polarized radiation of vertical polarization orientation, iv) the polarized radiation emitted by the at least one radiation source of the radiation arrangement is of vertical polarization orientation and the detector arrangement is configured to detect intensity of polarized radiation of vertical polarization orientation, v) the polarized radiation emitted by the at least one radiation source of the radiation arrangement is of vertical polarization orientation and the detector arrangement is configured to detect intensity of polarized radiation of horizontal polarization orientation, and vi) the polarized radiation emitted by the at least one radiation source of the radiation arrangement is of vertical polarization orientation and the detector arrangement is configured to detect both intensity of polarized radiation of vertical polarization orientation and intensity of polarized radiation of horizontal polarization orientation.

As suggested in the foregoing, it may be so that the UV radiation is the polarized radiation, or that another type of radiation is the polarized radiation. The first option implies that it is possible to have an embodiment of the system in which the at least one UV radiation source is the at least one radiation source of the radiation arrangement that is configured to emit the polarized radiation, whereas the second option implies that it is possible to have an embodiment of the system in which the radiation arrangement includes both the at least one UV radiation source and at least one additional radiation source configured to emit another type of radiation than UV radiation, wherein the at least one additional radiation source is the at least one radiation source of the radiation arrangement that is configured to emit the polarized radiation. The other type of radiation may particularly be radiation of distinctly longer wavelengths than UV wavelengths, such as visible light or infrared radiation. In view of the fact that absorption of radiation of longer wavelengths in the radiation body is typically significantly lower than the absorption of the UV radiation, detection sensitivity may be expected to be even higher when the detection functionality is based on the other type of radiation.

In a practical embodiment of the system according to the invention, the at least one radiation source of the radiation arrangement that is configured to emit the polarized radiation includes an assembly of a device configured to generate radiation and a polarizing device that is arranged in a radiation path of the device and configured to only let radiation of a predetermined polarization orientation pass through. For example, the polarizing device may comprise one of i) polarizer and ii) a polarizing beam splitter in combination with a half-wave plate. In the latter case, the half-wave plate serves to convert the polarization orientation of one part of the split beam into the polarization orientation of the other part of the split beam so that only a single polarization orientation results. Alternatively, the at least one radiation source of the radiation arrangement that is configured to emit the polarized radiation may comprise a suitable laser source, for example. Further, it may be practical if the detector arrangement comprises at least one assembly of a detector and a polarizing filter, so that the detector is only enabled to detect polarized radiation that is allowed to pass the filter.

The number of UV radiation sources and the number of detectors in the system can be chosen freely. Depending on the constitution of the radiation body and the size of the radiation exit window, it may be advantageous if the system comprises at least two UV radiation sources arranged in the radiation body at a distance from each other and/or if the system comprises at least two detectors arranged in the radiation body at a distance from each other. It may be practical if the radiation body comprises a slab of material, wherein the at least two UV radiation sources and/or the at least two detectors are embedded in the slab of material. The material of the slab of material may be silicone, for example. The at least one UV radiation source may be an LED, particularly a UV-C LED, for example.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

The above-described and other aspects of the invention will be apparent from and elucidated with reference to the following detailed description of practical embodiments of a system that is covered by the following definition: a system comprising i) a radiation body comprising a radiation exit window, wherein the radiation body is configured to receive radiation that at least comprises UV radiation, and wherein the radiation exit window is capable of allowing at least part of the radiation to pass to the exterior of the radiation body, ii) a radiation arrangement configured to provide the radiation, comprising at least one radiation source including at least one UV radiation source configured to emit UV radiation, wherein at least one radiation source of the radiation arrangement is configured to emit polarized radiation, iii) a detector arrangement configured to detect at least one of intensity of polarized radiation of the same polarization orientation as the polarized radiation emitted by the at least one radiation source and intensity of polarizated radiation of orthogonal polarization orientation, and iv) a controller arrangement, functionally coupled to the radiation arrangement and the detector arrangement.

### BRIEF DESCRIPTION OF THE DRAWINGS

The invention will now be explained in greater detail with reference to the figures, in which equal or similar parts are indicated by the same reference signs, and in which:
Fig. 1 illustrates an embodiment of a system according to the invention, and
Fig. 2 diagrammatically shows a number of objects which can be equipped with the system according to the invention.

### DETAILED DESCRIPTION OF THE EMBODIMENTS

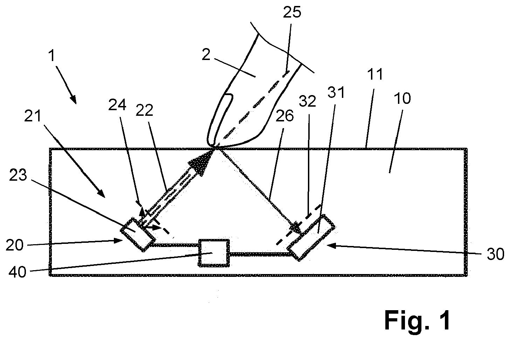

Fig. 1 illustrates an embodiment of a system 1 according to the invention.

The system 1 comprises a radiation body 10, a radiation arrangement 20, a detector arrangement 30 and a controller arrangement 40.

The radiation body 10 comprises a radiation exit window 11. Fig. 1 illustrates the practical options of the radiation body 10 comprising a slab of material and the radiation exit window 11 being of generally flat appearance.

The radiation arrangement 20 is configured to emit radiation that at least comprises UV radiation. In the shown example, the radiation arrangement 20 comprises a UV radiation source 21 configured to emit polarized UV radiation, in one or more directions, at least towards an interior side of the radiation exit window 11, wherein the polarized UV radiation is of either horizontal or vertical polarization orientation. In the following, for the sake of clarity, the polarization orientation of the polarized UV radiation emitted by the UV radiation source 21 will be referred to as source polarization orientation. In Fig. 1, the polarized UV radiation is depicted by means of an arrow 22. The UV radiation source 21 comprises a device 23 configured to generate UV radiation, such as a UV-C LED, and further comprises a polarizing device 24 that is arranged in a path of the UV radiation and configured to only let radiation waves of the UV radiation of the source polarization orientation pass through. This functionality of the polarizing device 24 is illustrated in Fig. 1, wherein two short arrows ending at the polarizing device 24 represent radiation waves which are not allowed to pass through. Further, Fig. 1 illustrates the practical option of the UV radiation source 21 being embedded in the slab of material of the radiation body 10, wherein the slab of material is transparent to the polarized UV radiation 22 provided by the UV radiation source 21. Any appropriate number of UV radiation sources 21 can be chosen in the context of the invention, wherein it is possible to have an arrangement of the UV radiation sources 21 in which the UV radiation sources 21 are positioned to radiate the polarized UV radiation 22 to respective areas of the radiation exit window 11 of the radiation body 10, whether or not with overlap.

The detector arrangement 30 is configured to detect intensity of polarized radiation of the source polarization orientation and/or intensity of polarized radiation of a polarization orientation that is orthogonal to the source polarization orientation. Fig. 1 illustrates the practical option of the detector arrangement 30 comprising a detector 31 and a polarizing filter 32 which are embedded in the slab of material of the radiation body 10. Any appropriate number of assemblies of a detector 31 and a polarizing filter 32 can be chosen in the context of the invention.

The controller arrangement 40 is functionally coupled to the radiation arrangement 20 and the detector arrangement 30 and is configured to control operation of the system 1. The invention covers both an option of the system 1 comprising a communication arrangement configured to enable data communication between the controller arrangement 40 and the radiation arrangement 20 and the detector arrangement 30, respectively, to take place in a wired fashion and an option of the system 1 comprising a communication arrangement configured to enable the data communication to take place in a wireless fashion, and also covers an option of the system 1 comprising a communication arrangement configured to enable the data communication to take place partly in a wired fashion and partly in a wireless fashion. Fig. 1 illustrates the practical option of the controller arrangement 40 comprising a unit that is embedded in the slab of material of the radiation body 10. That does not alter the fact that it is also possible that additionally or alternatively, the controller arrangement 40 comprises at least one unit that is positioned outside of the slab of material of the radiation body 10.

The radiation arrangement 20, the detector arrangement 30 and the controller arrangement 40 may be powered in any suitable way and may be electrically coupled to the mains or to a battery, for example.

The radiation exit window 11 of the radiation body 10 is configured to transmit part of the polarized UV radiation 22 of the UV radiation source 21. In Fig. 1, the radiation exiting the radiation body 10 through the radiation exit window 11 is indicated by means of a dashed line 25. The detector 31 is at a position of receiving internal radiation 26 emanating from the radiation exit window 11 inward into the slab of material of the radiation body 10, through the polarizing filter 32, and is configured to provide an output signal to the controller arrangement 40 representing intensity of part of the internal radiation 26, namely the part that is allowed to pass the polarizing filter 32. Depending on the structure of the radiation exit window 11, the internal radiation 26 may be obtained through at least one of i) scattering of the polarized UV radiation 22 radiated by the UV radiation source 21 at the position of the radiation exit window 11 and ii) reflection of the polarized UV radiation 22 radiated by the UV radiation source 21 on the interior side of the radiation exit window 11. It may be so that scattering of the polarized UV radiation 22 radiated by the UV radiation source 21 especially takes place when an object 2 such as a person's finger is present on an exterior side of the radiation exit window 11, as diagrammatically shown in Fig. 1. In any case, when the radiation exit window 11 is contacted by an object 2, both the exiting radiation 25 and the internal radiation 26 traveling from the interior side of the radiation exit window 11 towards the detector 31 change, both as far as intensity and polarization orientation are concerned.

The main purpose of having the radiation arrangement 20 in the system 1 is keeping the exterior side of the radiation exit window 11 in a disinfected state. When bacteria or viruses end up on the exterior side of the radiation exit window 11, the bacteria or viruses are killed or at least rendered inactive under the influence of the exiting radiation 25 at the position of the bacteria or viruses. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or animals/pets.

The controller arrangement 40 is configured to control the radiation arrangement 20 in dependence of the signal provided by the detector arrangement 30, especially when it comes to setting the intensity of the polarized UV radiation 22 emitted by the UV radiation source 21. The fact is that for safety reasons, it is desired to reduce the intensity of the radiation 22, probably to zero, in a situation of a person or animal/pet touching the radiation exit window 11. In view thereof, the controller arrangement 40 is configured to set a normal state of the UV radiation source 21 in which the UV radiation source 21 provides the polarized radiation 22 with an intensity at an operational level as a default, and to set an adapted state of the UV radiation source 21 in which the UV radiation source 21 provides the polarized UV radiation 22 with an intensity at a level that is reduced relative to the operational level after occurrence of a disturbance incident involving a change of the output signal of the detector 31. As explained earlier, this signal represents the intensity of the part of the internal radiation 26 that is allowed to pass the polarizing filter 32. In this respect, it is noted that occurrence of a disturbance incident is determined when a value of the intensity of the part of the internal radiation 26 shifts from inside a safety reference range of values to outside of the safety range of values.

In the case that the polarizing filter 32 is chosen such that the part of the internal radiation 26 that is allowed to pass is polarized radiation of the polarization orientation that is orthogonal to the source polarization orientation, the process of determining occurrence of a disturbance incident can be performed with high accuracy, because the fact is that the intensity and the polarization orientation of the internal radiation 26 in a situation of touch on the radiation exit window 11 are different from the intensity and the polarization orientation of the internal radiation 26 in the default situation. In the default situation, there is actually no polarized radiation of the polarization orientation that is orthogonal to the source polarization orientation, whereas in a situation of touch, the internal radiation 26 is of a different constitution and comprises the polarized radiation that is normally absent. This means that in a situation of touch, an output signal of the detector 31 is obtained that represents a significantly higher intensity of the polarized radiation of the polarization orientation that is orthogonal to the source polarization orientation. Contrariwise, in the case that the polarizing filter 32 is chosen such that the part of the internal radiation 26 that is allowed to pass is polarized radiation of the source polarization orientation, a situation of touch can be recognized on the basis of the fact that an output signal of the detector 31 is obtained that represents a lower intensity of the polarized radiation of the source polarization orientation.

In order to ensure proper functioning of the system 1, the references relied upon in assessing occurrence of a disturbance incident are preferably chosen such that occurrence of a disturbance incident is not determined when relatively small objects are present on the radiation exit window 11, particularly objects having dimensions of tens to hundreds micrometer or even smaller dimensions, such as tiny droplets and/or grease/dirt containing bacteria and/or viruses, but only when relatively large objects are present on the radiation exit window 11. The invention provides an uncomplicated and cost-effective way to combine the disinfection goal with a detection of possible presence of a higher organism, as a result of which the time that the system 1 is operated with the UV radiation source 21 in the default state is maximized and the risk of exposure of a higher organism to the UV radiation is minimized.

In general, the system 1 according to the invention may comprise any appropriate number of UV radiation sources 21 and any appropriate number of detectors 31. In a sophisticated embodiment, the detector arrangement 30 is configured to detect both intensity of polarized radiation of the source polarization orientation and intensity of polarized radiation of the polarization orientation that is orthogonal to the source polarization orientation. In such an embodiment, it is possible to keep track of a ratio of the respective intensities, and to involve in the process of determining occurrence of a disturbance incident an assessment of whether the ratio shifts from inside a safety reference range of ratios to outside of the safety range of ratios.

It is further to be noted that it is not necessary that the UV radiation is the polarized radiation. Alternatively, it is possible for the system 1 according to the invention to comprise both at least one UV radiation source 21 and at least one additional radiation source, wherein it is the at least one additional radiation source that is configured to emit polarized radiation. Such an at least one additional radiation source may be of the type that is configured to emit visible light, for example.

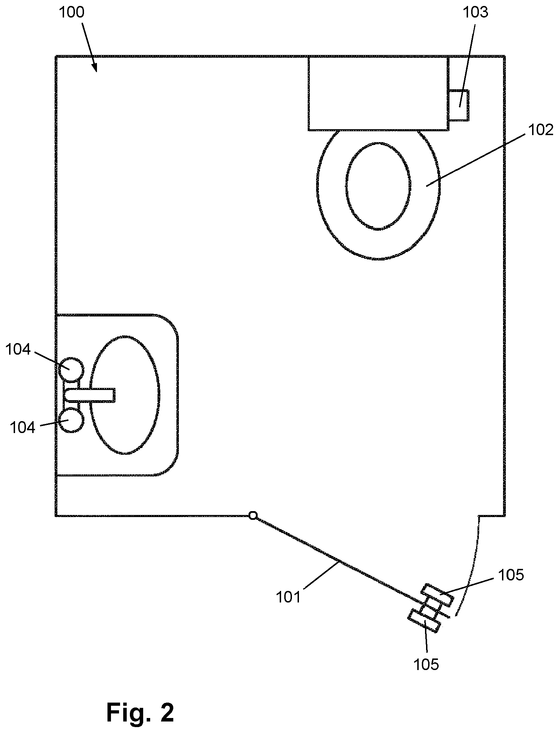

Fig. 2 diagrammatically shows a number of objects which can be equipped with the system 1 according to the invention, particularly objects as can be found in a bathroom 100 and on a bathroom door 101, namely a toilet seat 102, a toilet flushing knob 103, tap knobs 104 and the door knobs 105 of the bathroom door. The shown objects are only a few of the many objects included in the scope of protection of the invention. The object 102, 103, 104, 105 can be of conventional design, in which case the system 1 or at least the radiation body 10 of the system 1 and components arranged in the radiation body 10 can be arranged on an exterior surface of the object 102, 103, 104, 105, or the object 102, 103, 104, 105 can be of adapted design, in which case the system 1 or at least the radiation body 10 of the system 1 and components arranged in the radiation body 10 can have a sunk arrangement in a member of the object 102, 103, 104, 105, for example, wherein the radiation exit window 11 of the radiation body 10 of the system 1 can be flush with a surrounding part of an original exterior surface of the object 102, 103, 104, 105.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention are summarized as follows. In a system 1 comprising a radiation body 10, a radiation arrangement 20, a detector arrangement 30 and a controller arrangement 40, the radiation arrangement 20 serves for disinfection of a radiation exit window 11 of the radiation body 10. The controller arrangement 40 is configured to put at least one UV radiation source 21 of the radiation arrangement 20 from a default state to an adapted state of reduced radiation intensity when detection results obtained by means of the detector arrangement 30 demonstrate occurrence of a disturbance incident. In particular, at least one radiation source of the radiation arrangement 20, which may be the at least one UV radiation source 21, is configured to emit polarized radiation 22, and the detector arrangement 30 is configured to detect at least one of intensity of polarized radiation of the same polarization orientation as the polarized radiation 22 emitted by the at least one radiation source and intensity of polarizated radiation of orthogonal polarization orientation, wherein a change of the at least one of the intensities as mentioned is taken as an indication of occurrence of a disturbance incident. In this way, assuming that occurrence of a disturbance incident is indicative of the radiation exit window 11 being contacted by a relatively large object 2 such as a human hand or finger, the controller arrangement 40 is configured to enable disinfection of the radiation exit window 11 as a default, and to implement a safety measure of reducing the intensity at which UV radiation 22 is provided whenever appropriate.

## Claims

1. A system (1) comprising:
- a radiation body (10) comprising a radiation exit window (11), wherein the radiation body (10) is configured to receive radiation that at least comprises UV radiation, and wherein the radiation exit window (11) is capable of allowing at least part of the radiation to pass to the exterior of the radiation body (10);
- a radiation arrangement (20) configured to provide the radiation, comprising at least one radiation source (21) including at least one UV radiation source (21) configured to emit UV radiation (22), wherein at least one radiation source (21) of the radiation arrangement (20) is configured to emit polarized radiation (22);
- a detector arrangement (30) configured to detect at least one of intensity of polarized radiation of the same polarization orientation as the polarized radiation (22) emitted by the at least one radiation source (21) and intensity of polarized radiation of orthogonal polarization orientation; and
- a controller arrangement (40), functionally coupled to the radiation arrangement (20) and the detector arrangement (30), configured to set a normal state of the at least one UV radiation source (21) in which the at least one UV radiation source (21) provides the UV radiation (22) with an intensity at an operational level as a default, and to put the at least one UV radiation source (21) from the default state to an adapted state in which the at least one UV radiation source (21) provides the UV radiation (22) with an intensity at a level that is reduced relative to the operational level after occurrence of a disturbance incident involving a change of the at least one of intensity of polarized radiation of the same polarization orientation as the polarized radiation (22) emitted by the at least one radiation source (21) and intensity of polarized radiation of orthogonal polarization orientation detected by the detector arrangement (30).

2. The system (1) according to claim 1, wherein the controller arrangement (40) is configured to determine occurrence of a disturbance incident when a value of the at least one of intensity of polarized radiation of the same polarization orientation as the polarized radiation (22) emitted by the at least one radiation source (21) and intensity of polarized radiation of orthogonal polarization orientation detected by the detector arrangement (30) shifts from inside a safety reference range of values to outside of the safety range of values.

3. The system (1) according to claim 1 or 2, wherein the detector arrangement (30) is configured to detect both intensity of polarized radiation of the same polarization orientation as the polarized radiation (22) emitted by the at least one radiation source (21) and intensity of polarized radiation of orthogonal polarization orientation, and wherein the controller arrangement (40) is configured to determine occurrence of a disturbance incident when a ratio of the respective intensities shifts from inside a safety reference range of ratios to outside of the safety range of ratios.

4. The system (1) according to any of claims 1-3, wherein the at least one UV radiation source (21) is the at least one radiation source of the radiation arrangement (20) that is configured to emit the polarized radiation (22).

5. The system (1) according to any of claims 1-3, wherein the radiation arrangement (20) includes both the at least one UV radiation source (21) and at least one additional radiation source configured to emit radiation of distinctly longer wavelengths than UV wavelengths, and wherein the at least one additional radiation source is the at least one radiation source of the radiation arrangement that is configured to emit the polarized radiation.

6. The system (1) according to any of claims 1-5, wherein the at least one radiation source (21) of the radiation arrangement (20) that is configured to emit the polarized radiation (22) includes an assembly of a device (23) configured to generate radiation and a polarizing device (24) that is arranged in a radiation path of the device (23) and configured to only let radiation of a predetermined polarization orientation pass through.

7. The system (1) according to claim 6, wherein the polarizing device (24) comprises one of i) polarizer and ii) a polarizing beam splitter in combination with a half-wave plate.

8. The system (1) according to any of claims 1-5, wherein the at least one radiation source (21) of the radiation arrangement (20) that is configured to emit the polarized radiation (22) comprises a laser source.

9. The system (1) according to any of claims 1-8, wherein the detector arrangement (30) comprises at least one assembly of a detector (31) and a polarizing filter (32).

10. The system (1) according to any of claims 1-9, wherein the reduced level of the intensity of the UV radiation provided by the at least one UV radiation source (21) in the adapted state is a zero level.

11. The system (1) according to any of claims 1-10, wherein the radiation arrangement (20) comprises at least two UV radiation sources (21) arranged in the radiation body (10) at a distance from each other and/or wherein the detector arrangement (30) comprises at least two detectors (31) arranged in the radiation body (10) at a distance from each other.

12. The system (1) according to claim 11, wherein the radiation body (10) comprises a slab of material, and wherein the at least two UV radiation sources (21) and/or the at least two detectors (31) are embedded in the slab of material.

13. An object (102, 103, 104, 105) comprising the system (1) according to any of claims 1-12, wherein the radiation exit window (11) of the radiation body (10) of the system (1) is arranged to be at an exterior side of the object (102, 103, 104, 105).

14. The object (102, 103, 104, 105) according to claim 13, selected from a group comprising domestic appliances, furniture, construction elements of buildings and vehicles, sanitary parts, medical devices, and components of all of the foregoing.

15. Method of adding a system (1) according to any of claims 1-12 to an existing object (102, 103, 104, 105), wherein the radiation body (10) of the system (1) is applied to an exterior surface of the existing object (102, 103, 104, 105).
